# EUROPEAN PATENT SPECIFICATION

(11) **EP 0 376 912 B1**
(45) Date of publication and mention of the grant of the patent: **14.09.1994**
(21) Application number: 89870216.2
(22) Date of filing: 21.12.1989
(51) Int. Cl.: B01J 8/32, B01J 23/88, C07C 253/26

(54) **Fluid bed processes**
Wirbelbettverfahren
Procédés à lit fluidifié

(30) Priority: 29.12.1988 US 291892
(43) Date of publication of application: 04.07.1990
(73) Proprietor: MONSANTO COMPANY, St. Louis, Missouri 63167 (US)
(72) Inventor: Braun, John Ferrell, Houston Texas 77062 (US); Nowak, Robert Thomas, Webster Texas 77598 (US)
(74) Representative: Ernst, Hubert

(56) References cited:
- EP-A- 0 111 275
- FR-A- 2 242 143
- GB-A- 1 305 973
- US-A- 4 018 712
- US-A- 4 102 914
- US-A- 4 597 774

## Description

The present invention relates to improved fluid bed processes in which catalyst losses are significantly reduced. The invention is particularly advantageous in processes for ammoxidation of light hydrocarbons, preferably olefins, to unsaturated nitriles, such as acrylonitrile.

Many processes are known in which a bed of particulate catalyst is fluidized by a gaseous stream containing reactants which, in contact with the catalyst, are converted to desired reaction products. In such processes some catalyst, particularly smaller particle size catalyst, is entrained and must be separated from the product stream after it exits the fluid bed. This is frequently accomplished by cyclone means located downstream of the fluidized bed. Under the action of the cyclone, a major portion of the particulates are separated and recovered while the gaseous product stream passes overhead for further purification, utilization, or packaging. Unfortunately, during the process - most likely under severe conditions in the cyclone - a portion of the catalyst is converted to dust and exits the cyclone with the product stream and is thus considered lost. The problem is particularly apparent in ammoxidation processes where catalysts of relatively small particle sizes are frequently used. Such processes are exemplified, for example, in United States patents 3,164,626; 3,335,169; 3,446,834; 3,668,147; and 4,018,712; and 4,590,011. United States patent 4,590,011 teaches the admixture of inert particulate materials with active catalyst, such as described above, to improve product yield and inhibit formation of by products. GB-patent 1.305.973 relates to a process for controlling the fluidity of relatively coarse catalytic solids having generally below 5% of particles below 40 microns of size by providing, in the bed, a fine non-catalytic particle size fraction to thus re-establish a normal granulometry.

It has long been apparent that techniques for reducing solids losses in fluid bed processes would represent a significant advance in the art.

### SUMMARY OF THE INVENTION

The present invention resides in the discovery that catalyst losses in fluid bed reactors having cyclone means downstream of the fluidized bed are significantly reduced when the catalyst is mixed with a discrete, inert particulate material characterized by higher particle densities and lower particle sizes than the catalyst. In more precise terms, the invention herein relates to a fluidized bed catalytic reaction process wherein a gaseous feed stream is charged to a fluidized bed of particulate catalyst having a median particle size of 40-80 microns in a reactor system having cyclone means downstream of the fluidized bed for separation of entrained particles from a gaseous stream exiting the fluidized bed, the catalyst being mixed with a discrete inert particulate material present in an amount of from 5% to 40% by weight and having median particle sizes lower than the catalyst median particle size and densities higher than the catalyst density, characterized in that at least 20.5% by weight of the catalyst has a particle size of less than 44 microns and that the particle sizes, particle densities and quantities of inert particulates are selected such that particulates exiting the bottom of the last cyclone means in the reactor system comprise at least 5% by weight of said inert particulate material whereby catalyst losses are at least 25% lower than in an otherwise identical system containing no inert material.

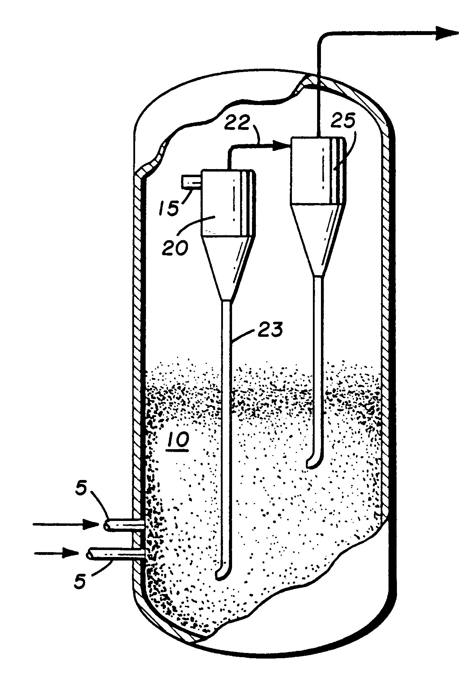

The invention will be understood from the following description of the preferred embodiments and the drawing in which FIGURE 1 is a schematic representation of a fluid bed reactor having associated cyclone means.

### DESCRIPTION OF THE PREFERRED EMBODIMENTS

The present invention is an improvement in fluidized bed processes of the type in which a gaseous feed stream containing reactants is charged to a fluidized bed of particulate catalyst in a reactor system having cyclone means downstream of the fluidized bed for separation of entrained particles from the gaseous stream exiting the fluidized bed.

The improvement is accomplished by use of a catalyst bed mixed with discrete, inert, particulate material. The term "discrete" is used to designate an inert material separate from and not being a part of the catalyst particle. That is, catalyst support material and deactivated catalyst (unless present as separate particles) are not considered as constituting any portion of the discrete inert particulate material. The term "inert" is used to designate materials which do not catalytically or chemically substantially participate in or adversely affect the fluid bed reaction in which they are utilized.

The particle sizes, particle densities and quantity of inert particulates are selected such that loss of catalyst is at least 25% by weight lower than in an otherwise identical system not containing the inert material. This result can usually be obtained, for example, by selection such that the particulates exiting the bottom (dip leg) of the last cyclone means of the reactor system (a reactor system may have one or a plurality of cyclone means) comprise at least 5% of catalyst weight, preferably at least 5-65%, of inert materials. By way of further example, use of from 5% to 40% of catalyst weight of inert materials having a median particle size no larger and preferably smaller than the largest particle size of the particles constituting the 25% by weight of catalyst of lowest particle size distribution is generally found to give acceptable results. (The term "median particle size" is used in the conventional sense as explained at pages 14, 15 Gas Fluidization Technology, D. Geldart - John Wiley, 1986 said disclosure being incorporated herein by reference.)

Inert particle densities higher than catalyst densities, preferably at least twice, and most preferably at least 2.3 times that of the catalyst are believed desirable to reduce catalyst losses.

Significant reductions in catalyst loss are generally achieved with 5% to 40% inert material. Higher quantities can be utilized but usually provide little additional reduction in catalyst loss. If desired, additional inert particles of sizes selected to promote optimum fluidization and/or achieve improved yields and inhibit formation of byproducts as taught in United States patent 4,590,011 can be utilized.

The inert particles and catalyst can be mixed before being charged to the reactor or can be separately charged or added in any order.

A representative fluid bed reactor system is illustrated in Figure 1 wherein gaseous reactants (diluted with inert diluents if desired) enter the system at opening 5; and pass through and fluidize a bed 10 of mixed catalyst and inert particles. Products, by products, unreacted reactants, and entrained particulates exit through conduit 15 into a first cyclone means 20 where a major portion of the particulates are separated and recovered at bottom exit 23. Exit gas and unseparated particulates flow through top exit 22 into a second cyclone 25 for further separation of gas and solids. The number of cyclones may be increased until further separation is not possible or is impractical. For purposes of this invention, the solids exiting the top of the lasts cyclone used in a system are considered "lost". Such solids are, in fact, considered lost in a practical sense in most commercial processes.

For purposes of specific description, the invention is hereinafter described with reference to its use in an ammoxidation process employing a catalyst disclosed in United States patent 4,018,712. It will be understood that the inventive concept is also applicable to other systems. The catalyst employed has the empirical formula

Sbₐ U_{b} Fe_{c} Bi_{d} Moₑ Me_{f} O_{g}

in which Mₑ is nickel or cobalt, a is 1 to 10, b is 0.1 to 5, c is 0.1 to 5, d is 0.001 to 0.1, e is 0.001 to 0.1, f is 0 to 0.1, and g is a number taken to satisfy the valences of the quantities of the other components present.

The inert material that is added can be any particulate which does not unduly interfere with the fluidizing properties of the catalyst used in the fluid bed, and which imparts no undesirable catalytic activity and has no undesirable chemical reactivity. Especially preferred is the use of low surface area alpha-alumina. The preferred physical properties of the alpha-alumina are set forth hereafter.

One convenient method of preparing the catalyst is to first combine the oxides, sulfates, or the like of antimony, uranium, iron, and bismuth with sulfuric acid. When antimony sulfate is used as a starting material, it can be added to water wherein sulfuric acid is obtained. Nitric acid is used to oxidize the sulfate salts of the various elements or to further oxidize the oxides of the elements. Instead of using sulfuric acid to digest the metal oxides, nitric acid can be used. After the acid mixture has digested, the pH of the mixture is adjusted to the basic side to cause precipitation of the oxides. The precipitate is then filtered. After the filtering operation, the filter cake is dried at a suitable temperature of from 100-180°C. A catalyst support may be added prior to or subsequent to drying. A suitable drying temperature is 110°C. However, drying can be obtained at higher temperatures such as up to 650°C. The time over which drying is accomplished can range from an hour up to 50 hours or more. Drying of the catalyst with or without the support can be advantageously accomplished, for example, by spray drying. The catalyst is calcined at a temperature in the range of 500 - 1150°C. The time of calcination can vary and depends upon the temperature employed. Generally, a time period of 1 - 24 hours at a selected temperature is sufficient. The calcination is preferred to be conducted in the presence of oxygen or air. The catalyst is shaped to suitable particle size having a desired surface area.

The inert material may be mixed with the catalyst before addition to the reaction zone or inert material and catalyst can be added separately. Preferably the catalyst has a surface area of 10-100 m²/g, a packed density of 0.9 - 1.1 g/ml, and an average particle size of 40-80 microns. At least 20.5% by weight of the catalyst has a particle size of less than 44 microns. The inert material has a surface area of less than 5m²/g, preferably of 0.5 - 3m²/g, a packed density of 0.5 to 2.5 g/l but preferably in the range of 0.9 - 2.5 g/ml, and a median particle size of less than 150 microns, preferably of 15-55 microns.

The catalyst mixed with the inert material exhibits utility in the conversion of olefins with or without the presence of ammonia. The olefins employed as reactants for the conversion by the catalyst may be open chain, as well as cyclic and include, for example, propylene, butene-1, butene-2, isobutene, pentene-1 pentene-2, 3-methyl butene-1, 2-methyl butene-2, hexene-1, hexene-2, 4-methyl pentene-1, 3,3-dimethylbutene-1, 4-methyl pentene-2, octene-1, cyclopentene, cyclohexene and the like. Of course, mixtures of olefins and mixtures of olefins with other hydrocarbons may be employed. When the catalyst and inert mixture of the present invention is to be used for ammoxidation, the olefins mentioned above are applicable. However, the system exemplified is particularly adapted to the conversion of propylene with ammonia and oxygen to acrylonitrile at 250 - 650°C.

The molar ratio of oxygen to the olefin in the feed will generally be in the range of 0.5:1 to 4:1 with a preferred ratio being 1:1 to 3:1. The molar ratio of ammonia to olefin in the feed will generally be in the range of 0.5:1 to 5:1 and preferably slightly over the stoichiometric ratio of 1:1 ammonia:olefin will be employed.

While ammonia is most generally employed as the nitrogen providing compound, other nitrogen containing materials may be employed which chemically change to produce reactive nitrogen under the selected reaction conditions. Any source of oxygen, pure or in admixture with inert gases, may be employed in the process of the invention. Air is a suitable source of oxygen.

### Example 1

Inert fines (white, fused, blocky shaped, 99.5% by weight, alpha aluminum oxide particles) having a particle size distribution as shown in Table 1, below, are added to a fluid bed reactor containing ammoxidation catalyst having the empirical formula Sb₁U_{0.18}Fe_{0.37}Bi_{0.01}Mo_{0.02}O_{sufficient} on 50% by weight SiO₂ support. Adjustments are made to the catalyst and inert fines inventory in the reactor to obtain a desired propylene conversion and provide the amounts of each shown in Table 1 below. The catalyst has a packed density (determined by addition of a weighed quantity of catalyst to a graduated cylinder and tapping the cylinder until no further reduction in volume is observed) of .98 gm/ml and the inert fines a packed density of 2.2 gm/ml. Particle density of the catalyst is 1.6 gm/ml and that of the inert fines is 3.9 gm/ml.

A reaction mixture of propylene, air and ammonia is passed through the reactor at the velocity indicated in the Table.

The exit stream from the reactor is divided and passed through separate sets of three cyclones in series. Total solids losses from the system and losses of inert fines are measured and are shown in Table 1 in which all percentages are by weight unless otherwise indicated.

**Table 1**

| Particle Size Distributions | | |
|---|---|---|
| Size | CATALYST Cumulative weight less than size indicated | INERT FINES Cumulative weight less than size indicated |
| 176.0 | 100.0 | 100.0 |
| 125.0 | 99.6 | 100.0 |
| 88.0 | 88.0 | 99.7 |
| 62.0 | 55.0 | 95.9 |
| 44.0 | 20.5 | 79.1 |
| 31.0 | 2.0 | 45.6 |
| 22.0 | 0.0 | 17.8 |
| 16.0 | 0.0 | 5.5 |
| 11.0 | 0.0 | 2.2 |
| 7.8 | 0.0 | 1.2 |
| 5.5 | 0.0 | 0.3 |
| 3.9 | 0.0 | 0.3 |
| 2.8 | 0.0 | 0.3 |
| median particle size | 59.4 microns | 32.8 microns |

### EXAMPLE I

| | Run 1 | Run 2 | Run 3 | Run 4 | Run 5 | Run 6 |
|---|---|---|---|---|---|---|
| Catalyst charge, kg | 17450 | 16100 | 17500 | 16770 | 14000 | 12800 |
| Inert fines charge, kg | 0 | 0 | 0 | 1266 | 5756 | 6759 |
| Fluid Bed Inert Fines % | 0 | 0 | 0 | 7 | 29 | 35 |
| Final Cyclone Inert Fines % | 0 | 0 | 0 | nd* | nd | 63 |
| Velocity, m/sec | 0.57 | 0.59 | 0.62 | 0.59 | 0.59 | 0.60 |
| Reactor Temperature, C | 464 | 464 | 461 | 464 | 463 | 464 |
| Reactor Pressure, atmosphere | 1.95 | 2.06 | 2.05 | 1.97 | 1.99 | 2.02 |
| Total Solids Loss, kg/day | 14.1 | 16.6 | 27.1 | 3.2 | 3.6 | 6.8 |
| % of loss that is inert fines | 0 | 0 | 0 | nd | nd | 47 |

| | | | | | | |
|---|---|---|---|---|---|---|
| *not determined | | | | | | |

### EXAMPLE 2

The test of Example 1 is repeated using catalyst and fines having particle size distributions as shown in Table 2.

**Table 2**

| Particle Size Distributions | | |
|---|---|---|
| Size | CATALYST Cumulative weight % less than size indicated | INERT FINES Cumulative weight % less than size indicated |
| 176.0 | 100.0 | 100.0 |
| 125.0 | 98.5 | 100.0 |
| 88.0 | 88.0 | 99.6 |
| 62.0 | 57.1 | 97.0 |
| 44.0 | 20.5 | 81.6 |
| 31.0 | 3.8 | 48.5 |
| 22.0 | 3.2 | 19.2 |
| 16.0 | 1.9 | 6.3 |
| 11.0 | 0.0 | 3.7 |
| 7.8 | 0.0 | 1.9 |
| 5.5 | 0.0 | 1.1 |
| 3.9 | 0.0 | 0.6 |
| 2.8 | 0.0 | 0.2 |
| median particle size | 58.5 microns | 31.6 microns |

### EXAMPLE 2

| | Run 7 | Run 8 | Run 9 | Run10 | Run11 | Run12 | Run13 | Run14 |
|---|---|---|---|---|---|---|---|---|
| Catalyst charge, kg | 19000 | 18500 | 18300 | 17700 | 17100 | 16900 | 17400 | 17200 |
| Inert fines, charge kg | 0 | 0 | 0 | 0 | 2164 | 1987 | 1814 | 1406 |
| Fluid Bed Inert Fines % | 0 | 0 | 0 | 0 | 11 | 10.5 | 9.5 | 7.6 |
| Final Cyclone Inert Fines, % | 0 | 0 | 0 | 0 | nd* | nd | nd | 5 |
| Velocity m/sec | 0.51 | 0.54 | 0.53 | 0.58 | 0.55 | 0.53 | 0.60 | 0.58 |
| Reactor Temp. C | 459 | 460 | 458 | 461 | 458 | 458 | 457 | 457 |
| Reactor Pressure atmospheres | 2.14 | 2.18 | 2.10 | 2.06 | 2.15 | 2.16 | 1.99 | 2.03 |
| Total Solids Loss kg/day | 7.3 | 7.3 | 5.2 | 17.2 | 3.6 | 0.6 | 5.9 | 4.1 |
| % of loss that is inert fines | 0 | 0 | 0 | 0 | nd | nd | nd | 11 |

| | | | | | | | | |
|---|---|---|---|---|---|---|---|---|
| *not determined | | | | | | | | |

It is seen in both examples that catalyst losses and total solids losses are significantly reduced. Acrylonitrile yields and conversions are not adversely affected.

## Claims

1. A fluidized bed catalytic reaction process wherein a gaseous feed stream is charged to a fluidized bed of particulate catalyst having a median particle size of 40-80 microns in a reactor system having cyclone means downstream of the fluidized bed for separation of entrained particles from a gaseous stream exiting the fluidized bed, the catalyst being mixed with a discrete inert particulate material present in an amount of from 5% to 40% by weight and having median particle sizes lower than the catalyst median particle size and densities higher than the catalyst density, characterized in that at least 20.5% by weight of the catalyst has a particle size of less than 44 microns and that the particle sizes, particle densities and quantities of inert particulates are selected such that particulates exiting the bottom of the last cyclone means in the reactor system comprise at least 5% by weight of said inert particulate material whereby catalyst losses are at least 25% lower than in an otherwise identical system containing no inert material.

2. The process of claim 1 wherein the catalytic reaction process is a process for ammoxidation of propylene to acrylonitrile in which a gaseous feed stream comprising propylene, ammonia and oxygen is charged to a fluidized bed containing particulate ammoxidation catalyst and is reacted to form acrylonitrile.

3. The process of claim 2 wherein the ammoxidation catalyst is represented by the formula
Sbₐ U_{b} Fe_{c} Bi_{d} Moₑ Me_{f} O_{g}
wherein Me is nickel or cobalt, a is 1 to 10, b is 0.1 to 5, c is 0.1 to 5, d is 0.001 to 0.1, e is 0.001 to 0.1, f is 0 to 0.1 and g is a number taken to satisfy the quantities and valences of Sb, U, Fe Bi, Mo and Me present.

4. The process of claim 1 further characterized in that the inert, particulate material has a particle density at least twice that of the catalyst and a mean particle size no greater than the largest particle size of the fourth of the catalyst having the smallest particle sizes.

5. The process of claim 1 wherein the catalytic reaction process is a process for ammoxidation of olefins in which a gaseous feed stream comprising olefins, ammonia and oxygen is charged to a fluidized bed containing particulate ammoxidation catalyst and is reacted to form unsaturated nitriles.

## Patentansprüche

1. Katalytisches Wirbelbett-Reaktionsverfahren, bei dem ein gasförmiger Speisestrom einem Wirbelbett aus einem teilchenförmigen Katalysator mit einer mittleren Teilchengröße von 40-80 µm aufgegeben wird, in einem Reaktorsystem mit einer Zykloneinrichtung stromabwärts des Wirbelbetts zur Abtrennung mitgeschleppter Teilchen aus einem gasförmigen Strom, welcher aus dem Wirbelbett austritt, wobei der Katalysator mit einem diskreten, inerten, teilchenförmigen Material vermischt wird, welches in einer Menge von 5 bis 40 Gew.-% vorliegt und mittlere Teilchengrößen, die geringer sind als die mittlere Teilchengröße des Katalysators und Dichten, welche höher sind als die Katalysatordichte, aufweist, **dadurch gekennzeichnet**, daß mindestens 20,5 Gew.-% des Katalysators eine Teilchengröße von weniger als 44 µm besitzen und daß die Teilchengrößen, Teilchendichten und Mengen inerter Teilchen so gewählt werden, daß Teilchen, welche aus dem Boden der letzten Zykloneinrichtung in dem Reaktorsystem austreten, mindestens 5 Gew.-% des inerten, teilchenförmigen Materials umfassen, wodurch Katalysatorverluste um mindestens 25% geringer sind als in einem ansonsten identischen System, welches kein inertes Material enthält.

2. Verfahren nach Anspruch 1, wobei das katalytische Reaktionsverfahren ein Verfahren zur Ammonoxidation von Propylen zu Acrylnitril ist, bei welchem ein Propylen, Ammoniak und Sauerstoff umfassender, gasförmiger Speisestrom einem Wirbelbett, welches teilchenförmigen Ammonoxidationskatalysator enthält, aufgegeben und zur Bildung von Acrylnitril umgesetzt wird.

3. Verfahren nach Anspruch 2, wobei der Ammonoxidationskatalysator der Formel
Sbₐ Uₚ Fe_{c} Bi_{d} Moₑ Me_{f} O_{g}
entspricht, worin Me Nickel oder Kobalt ist, a 1 bis 10 ist, b 0,1 bis 5 ist, c 0,1 bis 5 ist, d 0.001 bis 0,1 ist, e 0,001 bis 0,1 ist, f 0 bis 0,1 ist und g eine Zahl ist, die so gewählt wird, daß sie den Mengen und Wertigkeiten der vorhandenen Sb, U, Fe, Bi, Mo und Me genügt.

4. Verfahren nach Anspruch 1, weiterhin **dadurch gekennzeichnet**, daß das inerte, teilchenförmige Material eine Teilchendichte, die mindestens das zweifache derjenigen des Katalysators ist, und eine mittlere Teilchengröße besitzt, welche nicht größer ist als die größte Teilchengröße des Viertels des Katalysators mit den kleinsten Teilchengrößen.

5. Verfahren nach Anspruch 1, wobei das katalytische Reaktionsverfahren ein Verfahren zur Ammonoxidation von Olefinen ist, bei welchem ein Olefine, Ammoniak und Sauerstoff umfassender, gasförmiger Speisestrom einem Wirbelbett, welches teilchenförmigen Ammonoxidationskatalysator enthält, aufgegeben und zur Bildung ungesättigter Nitrile umgesetzt wird.

## Revendications

1. Procédé de réaction catalytique en lit fluidisé dans lequel un courant d'amenée gazeux est chargé dans un lit fluidisé de catalyseur en particules présentant un taille moyenne de particule de 40-80 micromètres dans un système de réacteur comportant un moyen formant cyclone en aval du lit fluidisé pour séparer d'un courant gazeux sortant du lit fluidisé les particules entraînées, le catalyseur étant mélangé avec une matière inerte en particules présente en une quantité allant de 5% jusqu'à 40% en poids et présentant des tailles moyennes de particule inférieures à la taille moyenne de particule du catalyseur et des densités supérieures à la densité du catalyseur, caractérisé en ce qu'au moins 20,5% en poids du catalyseur présentent une taille de particule inférieure à 44 micromètres et que les tailles des particules, les densités des particules et les quantités des particules inertes sont choisies de manière que les particules sortant à la base du dernier moyen formant cyclone dans le système de réacteur comprennent au moins 5% en poids de ladite matière inerte en particules, grâce à quoi les pertes de catalyseur sont au moins 25% inférieures à celles d'un système par ailleurs identique mais ne contenant pas de matière inerte.

2. Procédé selon la revendication 1, dans lequel le procédé de réaction catalytique est un procédé de transformation du propylène en acrylonitrile par ammoxydation, dans lequel un courant d'amenée gazeux comprenant du propylène, de l'ammoniac et de l'oxygène est chargé dans un lit fluidisé contenant un catalyseur d'ammoxydation en particules et est amené à réagir pour former un acrylonitrile.

3. Procédé selon la revendication 2, dans lequel le catalyseur d'ammoxydation est représenté par la formule :
Sbₐ U_{b} Fe_{c} Bi_{d} Moₑ Me_{f} O_{g}
où Me est du nickel ou du cobalt, a est compris entre 1 et 10, b est compris entre 0,1 et 5, c est compris entre 0,1 et 5, d est compris entre 0,001 et 0,1, e est compris entre 0,001 et 0,1, f est compris entre 0 et 0,1 et g est un nombre pris pour satisfaire aux quantités et aux valences de Sb, U, Fe, Bi, Mo et Me présents.

4. Procédé selon la revendication 1, caractérisé en outre en ce que la matière inerte en particules présente une densité de particule égale à au moins deux fois celle du catalyseur et une taille moyenne de particule qui n'est pas supérieure à la taille maximale de particule du quatrième des catalyseurs ayant les tailles minimales de particules.

5. Procédé selon la revendication 1, dans lequel le procédé de réaction catalytique est un procédé pour ammoxydation des oléfines, dans lequel un courant d'amenée gazeux comprenant des oléfines, de l'ammoniac et de l'oxygène, est chargé dans un lit fluidisé contenant un catalyseur d'ammoxydation en particules et est amené à réagir de manière à former des nitriles insaturés.
